# EUROPEAN PATENT SPECIFICATION

(11) **EP 2 745 871 B1**
(45) Date of publication and mention of the grant of the patent: **03.05.2017**
(21) Application number: 13198214.2
(22) Date of filing: 18.12.2013
(51) Int. Cl.: A61M 25/09

(54) **Guide wire**
Führungsdraht
Fil-guide

(30) Priority: 20.12.2012 US 201261740202 P
(43) Date of publication of application: 25.06.2014
(73) Proprietor: Cook Medical Technologies LLC, Bloomington, IN 47404 (US)
(72) Inventor: Isch, Andrew P., Indiana 47906 (US)
(74) Representative: Garratt, Peter Douglas

(56) References cited:
- WO-A1-98/50103
- WO-A1-2004/093966
- US-A1- 2005 021 075
- US-B1- 6 533 772

## Description

### CROSS-REFERENCE TO RELATED APPLICATIONS

This application claims priority from United States Provisional Application No. 61/740,202, filed on December 20, 2012.

### TECHNICAL FIELD

This disclosure relates to medical devices and more particularly to a guide wire for placement of a tubular medical device within a body lumen.

### BACKGROUND

A guide wire is commonly used in a medical procedure to provide a pathway over which another medical device may be navigated. The pathway provided by the guide wire may be used to navigate the other medical device through a body vessel. Typically, the guide wire is navigated through a body vessel toward a point of treatment. Once positioned within the body vessel, a second medical device (e.g., a cannula or a catheter) may be advanced over the guide wire and moved along the length of the guide wire toward the point of treatment. The guide wire provides an established path for placing other medical devices, thus eliminating the need for performing delicate navigation procedures for each medical device introduced into the body vessel.

In one particular medical procedure, a guide wire is used for placement of a ureteral stent. Typically, a scope (e.g., a cystoscope, a uretheroscope, or a ureteroscope) is introduced through the urethra and into the bladder. The guide wire is introduced through the ureteral orifice in the bladder via the scope and advanced through the ureter and into the calyx of the kidney. The scope is removed over the guide wire, and the ureteral stent is introduced over the guide wire for placement within the ureter.

During such a procedure, the guide wire may be inadvertently removed from the patient. For example, the guide wire may fall out of the patient prior to introduction of the ureteral stent (e.g., during removal of the scope). In this instance, a physician may be required to reintroduce the scope into the bladder to replace the guide wire within the ureter. This adds to the time required to perform the medical procedure. To avoid such a situation, the physician may place a second guide wire prior to removing the scope from the patient. The second guide wire serves as a backup in case the first guide wire is inadvertently removed. This adds to the cost of the medical procedure because two guide wires are used instead of a single guide wire. Reference is directed to WO9850103 which discloses a percutaneous catheter including a filter for capturing loose embolic material used to deliver a stent. The catheter includes an expandable stent deployed on an inflatable balloon, or alternatively a self-expanding stent. A filter assembly comprises an expansion frame having filter mesh attached to it disposed on a guidewire inserted through the catheter, or sheath. The expansion frame comprises a structure which is activated from a contracted condition into an enlarged condition in a blood vessel, thereby traversing the vessel with the filter mesh. The expansion frame includes biased struts, a mechanically operated frame, and/or a fluid operated frame. Reference is also directed to WO2004093966 which discloses an expandable element guide wire having outer and inner guide wires. A braided or malecot type of expandable element is secured between the distal end of the outer guide wire and the distal end of the inner guide wire. Relative movement between the outer and inner guide causes the expandable element to move to a radially contracted condition. Reference is also directed to US 2005/0021075 which discloses a protective system or apparatus for use in vascular procedures including a tubular guidewire, a control cable slidable within the tubular guidewire, and a sheathless filter. The control cable is attached to a distal end of the sheathless filter and the tubular guidewire is attached to a proximal end of the sheathless filter. Selective displacement of the control cable radially expands the sheathless filter to create a proximal exterior convex primary filter surface that is positionable downstream from a site of a vascular procedure. The sheathless filter also presents a distal interior concave secondary filter surface. Preferably, the sheathless filter is constructed of a braided wire framework in the form of a tube over which woven polymer fibers or strands are applied to create a filter mesh having a softer filter surface. Reference is also directed to US 6533772 which discloses a guide wire torque device having an elongate tubular body with an interior long axial channel and a rotary clamp wheel for gripping and ungripping a guide wire extending through the channel. The clamp wheel is accommodated within an integral enlargement of the tubular body with a segment of the wheel surface extending through the surface of the enlargement for rotation by a user's thumb in order to grip and ungrip the guide wire for feeding, withdrawing or rotating the wire with respect to a catheter.

### SUMMARY

The present embodiments provide a guide wire for placement of a tubular medical device within a body lumen.

In one example, a guide wire may include a core member about a longitudinal axis of the guide wire and having a distal end. An outer coil may be disposed about the core member and include a proximal end and a distal end disposed proximal of the distal end of the core member. The outer coil may extend longitudinally along a majority of a length of the guide wire. The outer coil may be longitudinally movable relative to the core member between a retracted position and an advanced position. The distal end of the outer coil may be longitudinally spaced from the distal end of the core member by a greater distance in the retracted position than in the advanced position. An anchor member may include an elongate wire extending longitudinally between the distal end of the outer coil and the distal end of the core member. The anchor member may have a proximal end attached to the distal end of the outer coil and a distal end attached to the distal end of the core member. In response to longitudinal movement of the outer coil relative to the core member, the anchor member may be movable between a delivery configuration and a deployed configuration. At least a portion of the anchor member may be positioned radially away from the longitudinal axis of the guide wire by a greater distance in the deployed configuration than in the delivery configuration. A retaining mechanism may include a tubular member removably positionable about the core member and in abutting contact with a proximal end of the outer coil. The retaining mechanism may be engageable with the core member to inhibit longitudinal movement of the outer coil proximally relative to the core member.

In another example, a guide wire may include a core member about a longitudinal axis of the guide wire and having a proximal end and a distal end. An outer coil may be disposed about the core member and include a proximal end and a distal end. Each of the proximal end and the distal end of the outer coil may be disposed between the proximal end and the distal end of the core member. The outer coil may be longitudinally movable along the core member between a retracted position and an advanced position. The distal end of the outer coil may be farther from the distal end of the core member in the retracted position than in the advanced position. An anchor member may include an elongate wire extending longitudinally between the distal end of the outer coil and the distal end of the core member. The anchor member may have a proximal end fixedly attached to the distal end of the outer coil, a distal end fixedly attached to the distal end of the core member, and an intermediate portion between the proximal end and the distal end of the anchor member. In response to longitudinal movement of the outer coil relative to the core member, the anchor member may be movable between a delivery configuration in which the intermediate portion of the anchor member is substantially linear and a deployed configuration in which the intermediate portion of the anchor member is bowed outward away from the longitudinal axis of the guide wire. A retaining mechanism may include a tubular member removably disposed about the core member and in abutting contact with the proximal end of the outer coil. The retaining mechanism may be movable between a released configuration in which the core member is slidable within the retaining mechanism and an engaged configuration in which the retaining mechanism is frictionally engaged with the core member to inhibit longitudinal movement of the outer coil relative to the core member.

In another example, a guide wire may comprise: a core member about a longitudinal axis of the guide wire and comprising a proximal end and a distal end; an outer coil disposed about the core member and comprising a proximal end and a distal end, each of the proximal end and the distal end of the outer coil disposed between the proximal end and the distal end of the core member, the outer coil being longitudinally movable along the core member between a retracted position and an advanced position, the distal end of the outer coil being farther from the distal end of the core member in the retracted position than in the advanced position; an anchor member comprising an elongate wire extending longitudinally between the distal end of the outer coil and the distal end of the core member and comprising a proximal end fixedly attached to the distal end of the outer coil, a distal end fixedly attached to the distal end of the core member, and an intermediate portion between the proximal end and the distal end of the anchor member, whereby, in response to longitudinal movement of the outer coil relative to the core member, the anchor member is movable between a delivery configuration in which the intermediate portion of the anchor member is substantially linear and a deployed configuration in which the intermediate portion of the anchor member is bowed outward away from the longitudinal axis of the guide wire; and a retaining mechanism comprising a tubular member removably disposed about the core member and in abutting contact with the proximal end of the outer coil, the retaining mechanism being movable between a released configuration in which the core member is slidable within the retaining mechanism and an engaged configuration in which the retaining mechanism is frictionally engaged with the core member to inhibit longitudinal movement of the outer coil relative to the core member. The anchor member may be biased toward the delivery configuration, whereby the outer coil is biased toward the retracted position. The anchor member may comprise a plurality of anchor members disposed about the core wire. In response to movement of the outer coil from the retracted position toward the advanced position, the proximal end of each anchor member and the distal end of each anchor member may be moved longitudinally toward one another, and a loop is formed in the intermediate portion of each anchor member. The guide wire may further comprise a tip positioned at the distal end of the core member, wherein the distal end of each anchor member is attached to the distal end of the core member via the tip. The retaining mechanism may comprise a proximal portion and a distal portion, and the proximal portion is rotatable relative to the distal portion to move the retaining mechanism between the released configuration and the engaged configuration.

In another example, a method of placing a tubular medical device within a body lumen may include introducing a guide wire through a scope and into the body lumen. The guide wire may include a core member, an outer coil disposed about the core member, and an anchor member including an elongate wire having a proximal end attached to a distal end of the outer coil and a distal end attached to a distal end of the core member. The anchor member may be moved from a delivery configuration toward a deployed configuration by moving the outer coil distally over the core member to move the proximal end of the anchor member distally toward the distal end of the anchor member. An intermediate portion of the anchor member may bow radially outward away from the core member. A retaining mechanism may be positioned about the core member and in abutting contact with a proximal end of the outer coil disposed external of the body lumen. The retaining mechanism may be moved from a released configuration to an engaged configuration. The retaining mechanism may frictionally engage the core member to fix the outer coil in place relative to the core member to retain the anchor member in the deployed configuration.

The scope may be retracted proximally over the guide wire with the anchor member remaining in the deployed configuration and the retaining mechanism remaining positioned about the core member and in the engaged configuration. The anchor may be positioned in a body cavity in fluid communication with the body lumen and moving the anchor member from the delivery configuration toward the deployed configuration with the anchor member in the body cavity, wherein an outer diameter of the anchor member in the deployed configuration is larger than an inner diameter of the body lumen to inhibit the guide wire from moving proximally within the body lumen. The body cavity may comprise a kidney, and the body lumen a ureter. The retaining mechanism may be moved from the engaged configuration to the released configuration, removing the retaining mechanism from the core member by retracting the retaining mechanism proximally over a proximal end of the core member, and advancing the medical device distally over the guide wire into the body lumen subsequent to removing the retaining mechanism from the core member. The retaining mechanism may comprise an outer diameter that is larger than an inner diameter of the medical device.

Other systems, methods, features, and advantages of the invention will be, or will become, apparent to one with skill in the art upon examination of the following figures and detailed description. It is intended that all such additional systems, methods, features, and advantages be within the scope of the invention, and be encompassed by the following claims.

### BRIEF DESCRIPTIONS OF THE DRAWINGS

FIG. 1 illustrates one example of a guide wire having a plurality of anchor members in a delivery configuration.
FIG. 2 illustrates the guide wire of FIG. 1 with the plurality of anchor members in a deployed configuration.
FIG. 3 illustrates a proximal portion of one example of a guide wire having a plurality of anchor members in a delivery configuration.
FIG. 4 illustrates the proximal portion of the guide wire of FIG. 3 with the plurality of anchor members in a deployed configuration.
FIG. 5 illustrates one example of a retaining mechanism.
FIG. 6 illustrates one example of a retaining mechanism.
FIG. 7 illustrates the guide wire of FIGS. 1-2 deployed within a ureter.

### DETAILED DESCRIPTION OF THE DRAWINGS AND THE PRESENTLY PREFERRED EMBODIMENTS

The present disclosure relates to a guide wire for placement of a tubular medical device within a body lumen. The embodiments described in this disclosure will be discussed generally in relation to a guide wire for placement of a ureteral stent within a ureter, but the disclosure is not so limited. The guide wire may be used to place any type of medical device capable of introduction over the guide wire within any body lumen, body vessel, and/or body cavity. For example, the guide wire may be used for placement of a ureteral stent, a urethral stent, a biliary stent, or a gastrointestinal stent.

In the present disclosure, the term "proximal" refers to a direction that is generally toward a physician during a medical procedure, while the term "distal" refers to a direction that is generally toward a target site within a patient's anatomy during a medical procedure.

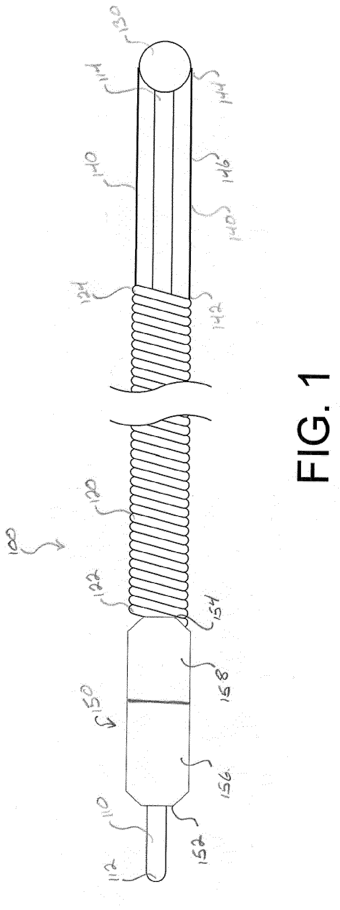

**FIG. 1** illustrates one example of a guide wire 100. The guide wire 100 may include a core member 110 and an outer sleeve 120 disposed about the core member. A tip 130 may be positioned at a distal end of the core member 110. One or more anchor members 140 may extend longitudinally between the outer sleeve 120 and the tip 130. The anchor members 140 may be movable between a delivery configuration and a deployed configuration to aid in retaining the guide wire 100 in place within a body vessel as further described below. A retaining mechanism 150 may be positioned about the core member 110 at a proximal end of the outer sleeve 120. The retaining mechanism 150 may aid in retaining the outer sleeve 120 in position relative to the core member 110 as further described below.

The core member 110 may be configured as an elongate member having a proximal end 112 and a distal end 114. The core member 110 may be configured to enhance the longitudinal stability and/or pushability of the guide wire 100. To that end, the core member 110 may have a relatively high stiffness. For example, the core member 110 may be configured as a solid wire. Alternatively, the core member 110 may be configured as a tubular member (e.g., a cannula or a hypotube). The core member 110 may have sufficient radial flexibility to enable navigation of the guide wire 100 through relatively tortuous anatomy as further described below. The core member 110 may have any suitable cross sectional shape such as, for example, circular, elliptical, triangular, rectangular, or any other polygonal or non-polygonal shape.

The outer sleeve 120 may be disposed about the core member 110. To that end, the outer sleeve 120 may be configured as an elongate tubular member having a proximal end 122, a distal end 124, and a lumen extending longitudinally within the outer sleeve. The outer sleeve 120 may be configured to enhance the kink-resistance and/or radial flexibility of the guide wire 100. To that end, the outer sleeve 120 may be configured as a coiled member or an outer coil having a sidewall formed from a series of windings as shown in **FIG. 1****.** The coiled member may be configured as a continuous coiled wire wrapped around the core member 110. Alternatively, the coiled member may be formed from a plurality of coiled wires joined to one another to collectively form the coiled member. The coiled member may extend longitudinally along a majority of the length of the guide wire 100. In this manner, the guide wire may be provided with kink-resistance and/or radial flexibility along the majority of the length of the guide wire 100 (e.g., the portion of the guide wire that may be introduced into a body vessel during a medical procedure) to aid in navigating the guide wire through a body vessel. In other examples, the outer sleeve 120 may be configured as a tubular member having a substantially solid sidewall (e.g., a cannula or a hypotube).

The coiled member may be formed using any known technique including, for example, wrapping one or more wires around a mandrel. The wires may have any suitable cross sectional shape such as, for example, circular, elliptical, triangular, rectangular, or any other polygonal or non-polygonal shape. In one example, a low profile coil, such as a coil having a conventional flat wire construction, may be used to minimize the cross sectional profile (i.e., the outer diameter) of the outer sleeve 120. Adjacent windings of the coiled member may be in abutting contact with one another as shown in **FIG. 1****.** In this manner, the coiled member may be substantially longitudinally incompressible. This may enhance the longitudinal stability and/or pushability of the guide wire 100. Additionally, or alternatively, the adjacent windings may be capable of moving relative to one another in a radial direction and/or a longitudinal direction. This may enhance the flexibility of the coiled member, which may enhance the flexibility of the guide wire 100.

The core member 110 may be received within the lumen of the outer sleeve 120 as shown in **FIG. 1****.** The core member 110 may be longer than the outer sleeve 120 such that the core member extends beyond the ends of the outer sleeve. For example, the core member 110 may extend proximally beyond the proximal end 122 of the outer sleeve 120. In other words, the proximal end 112 of the core member 110 may be disposed proximal of the proximal end 122 of the outer sleeve 120. Additionally, or alternatively, the core member 110 may extend distally beyond the distal end 124 of the outer sleeve 120. In other words, the distal end 114 of the core member 110 may be disposed distal of the distal end 124 of the outer sleeve 120.

The core member 110 and the outer sleeve 120 may be formed from any suitable material known in the art. Suitable materials may include, for example, metallic or polymeric materials. The core member 110 and the outer sleeve 120 may be formed from the same or different materials. In one example, the core member 110 and the outer sleeve 120 may be formed from a metallic material such as nitinol, stainless steel, platinum, or palladium. The use of platinum and/or palladium may enhance the radiopacity of the guide wire 100. Additionally, or alternatively, the core member 110 and/or the outer sleeve 120 may be coated with a lubricious, low friction, and/or non-stick material (e.g., polytetrafluoroethylene (PTFE), sodium bicarbonate, a silicone lubricant, or any other biocompatible lubricant). This may aid in passing an interventional medical device over the outer sleeve 120 and/or moving the outer sleeve relative to the core member 110 as described herein.

The tip 130 may be positioned at the distal end 114 of the core member 110. The tip 130 may be configured as an atraumatic tip. In other words, the tip 130 may have a blunt or rounded shape, which may aid in preventing damage to an inner wall of a body vessel during introduction of the guide wire 100 as further described below. In one example, the tip 130 may be configured as a rounded solder at the distal end 114 of the core member 110 as shown in **FIG. 1****.** Additionally, or alternatively, the tip may be configured as a flexible or floppy tip extending distally from the distal end 114 of the core member 110. The flexible tip may be configured, for example, as described in U.S. Patent No. 5,234,003 to Hall. Additionally, or alternatively, the tip may be configured as a shaped tip (e.g., curved, looped, J-shaped, or otherwise shaped) extending distally from the distal end 114 of the core member 110. The tip 130 may include a radiopaque material to enable imaging of the tip during a medical procedure.

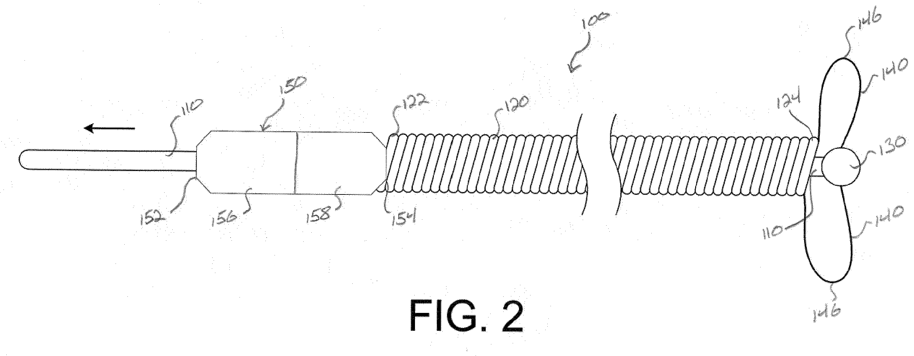

The outer sleeve 120 may be longitudinally movable relative to the core member 110 between a retracted position as shown in **FIG. 1** and an advanced position as shown in **FIG. 2****.** In the retracted position, the distal end 124 of the outer sleeve 120 may be spaced longitudinally from the distal end 114 of the core member 110 and/or the tip 130 by a first distance. In the advanced position, the distal end 124 of the outer sleeve 120 may be spaced longitudinally from the distal end 114 of the core member 110 and/or the tip 130 by a second distance. The first distance may be greater than the second distance. In other words, the distal end 124 of the outer sleeve 120 may be longitudinally spaced from the distal end 114 of the core member 110 and/or the tip 130 by a greater distance in the retracted position than in the advanced position as shown in **FIGS. 1-2****.** In one example, the first distance may be between about 13 mm (0.5 inches) and about 51 mm (2 inches). The outer sleeve 120 may be advanced distally relative to the core member 110 to move the outer sleeve from the retracted position toward the advanced position. Additionally, or alternatively, the core member 110 may be retracted proximally relative to the outer sleeve 120 to move the outer sleeve from the retracted position toward the advanced position. Such longitudinal movement may enable deployment of the anchor member 140 to aid in retaining the guide wire 100 in place within a body vessel as further described below.

The anchor member 140 may extend longitudinally between the distal end 124 of the outer sleeve 120 and the distal end 114 of the core member 110. Additionally, or alternatively, the anchor member 140 may extend longitudinally between the distal end 124 of the outer sleeve 120 and the tip 130. The anchor member 140 may be attached to the distal end 124 of the outer sleeve 120 and the distal end 114 of the core member 110. In this manner, the outer sleeve 120 may be coupled to the core member 110 via the anchor member 140. This may inhibit the outer sleeve 120 from moving rotationally relative to the core member 110. In other words, the outer sleeve 120 may be substantially non-rotatable relative to the core member 110. This may aid in applying a twisting force to the guide wire 100 for navigation through a body vessel. Additionally, or alternatively, longitudinal movement of the outer sleeve 120 in a proximal direction may be limited by the anchor member 140. For example, the outer sleeve 120 may be inhibited from moving proximally beyond the retracted position by the anchor member 140 in the delivery configuration. In this manner, the outer sleeve 120 may be non-removable from the core member 110.

In one example, the anchor member 140 may be attached to the distal end 114 of the core member 110 via the tip 130. To that end, the anchor member 140 may be attached to the distal end 124 of the outer sleeve 120 and the tip 130 as shown in **FIGS. 1-2****.** In this manner, the anchor member 140 may be unattached directly to the core member 110. In other words, the anchor member 140 may be independent of the core member 110. Alternatively, the anchor member 140 may be directly attached to the core member 110. The tip 130 may be omitted from examples in which the anchor member 140 is directly attached to the core member 110. In one example, the anchor member 140 may be soldered or welded to the core member 110, the outer sleeve 120, and/or the tip 130. In other examples, the anchor member 140 may be attached to the core member 110, the outer sleeve 120, and/or the tip 130 with any suitable attachment mechanism such as, for example, an adhesive, a bonding agent, a mechanical fastener (e.g., a screw or a bolt), or any other suitable attachment mechanism. Additionally, or alternatively, the anchor member 140 may be non-releasably attached to the core member 110, the outer sleeve 120, and/or the tip 130.

The anchor member 140 may be configured as an elongate member having a proximal end 142, a distal end 144, and an intermediate portion 146 between the proximal end and the distal end of the anchor member. The anchor member 140 may be configured as a length of wire or ribbon. For example, the anchor member 140 may be configured as a wire having a rectangular cross sectional shape (e.g., a flat wire). Alternatively, the anchor member 140 may be configured as a wire having any other suitable cross sectional shape such as, for example, circular, elliptical, triangular, or any other polygonal or non-polygonal shape. The proximal end 142 of the anchor member 140 may be attached to the distal end 124 of the outer sleeve 120. The distal end 144 of the anchor member 140 may be attached to the distal end 114 of the core member 110 and/or the tip 130. In this manner, the longitudinal position of the distal end 144 of the anchor member 140 may be fixed relative to the core member 110. In other words, the distal end 144 of the anchor member 140 may be incapable of moving longitudinally relative to the distal end 114 of the core member 110. The intermediate portion 146 of the anchor member 140 may be unattached directly to the core member 110, the outer sleeve 120, and/or the tip 130. In this manner, the intermediate portion 146 of the anchor member 140 may be radially movable (e.g., in a radial direction transverse to the longitudinal axis of the guide wire 100). For example, the intermediate portion 146 may be movable radially away from the core member 110, the outer sleeve 120, and/or the tip 130 to aid in retaining the guide wire 100 in place within a body vessel as further described below.

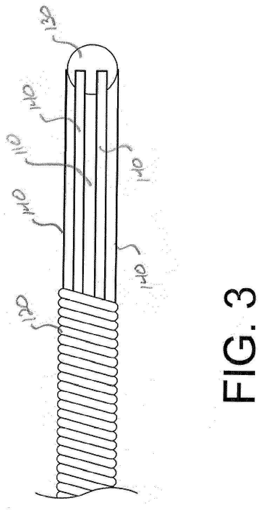

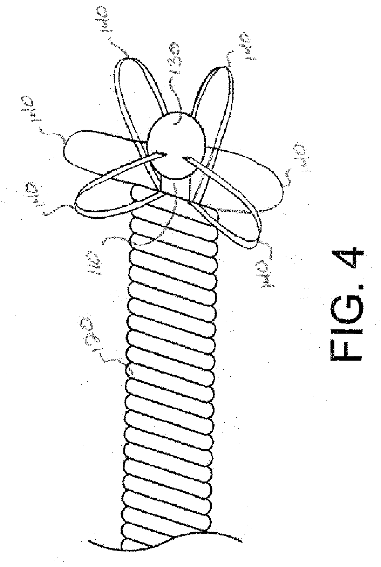

The guide wire 100 may include a plurality of anchor members 140 disposed radially about the core member 110. For example, the guide wire 100 may include two anchor members 140 as shown in **FIGS. 1-2****.** The anchor members 140 may be disposed diametrically opposite one another relative to the circumference of the core member 110. In other words, the two anchor members 140 may be circumferentially spaced from one another by about 180 degrees. In other examples, the guide wire may have any suitable number of anchor members (e.g., one, three, four, or more) disposed about the core member at any suitable circumferential positions. In one example, the guide wire may have six anchor members as shown in **FIGS. 3-4** and further described below. Typically, the guide wire may have between two and ten anchor members.

The anchor member 140 may be movable between a delivery configuration as shown in **FIG. 1** and a deployed configuration as shown in **FIG. 2****.** In the delivery configuration, a radially outermost portion of the anchor member 140 may be spaced from the longitudinal axis of the guide wire 100 by a first radial distance. In the deployed configuration, a radially outermost portion of the anchor member 140 may be spaced from the longitudinal axis of the guide wire 100 by a second radial distance. The second radial distance may be greater than the first radial distance. In other words, the radially outermost portion of the anchor member 140 may be positioned radially away from the longitudinal axis of the guide wire 100 by a greater distance in the deployed configuration than in the delivery configuration as further described below.

**FIG. 1** illustrates the guide wire 100 with the anchor member 140 in the delivery configuration. In the delivery configuration, the anchor member 140 may have a substantially linear configuration. In other words, the anchor member 140 may extend longitudinally in a substantially straight line between the proximal end 142 and the distal end 144 of the anchor member. The intermediate portion 146 of the anchor member 140 may be disposed adjacent to the core member 110. In other words, the intermediate portion 146 may be disposed radially in close proximity to and/or in abutting contact with the core member 110 as shown in **FIG. 1****.** An outer radius of the anchor member 140 may be the distance between the longitudinal axis of the guide wire 100 and the outermost portion of the anchor member 140. In the delivery configuration, the outer radius of the anchor member 140 may be less than or equal to an outer radius of the outer sleeve 120. Additionally, or alternatively, the outer radius of the guide wire 100 may be substantially uniform between the proximal end 122 of the outer sleeve 120 and the tip 130.

Longitudinal movement of the outer sleeve 120 distally relative to the core member 110 may cause a corresponding longitudinal movement of the proximal end 142 of the anchor member 140 toward the distal end of the anchor member 144. In other words, such movement of the outer sleeve 120 may cause the proximal end 142 and the distal end 144 of the anchor member 140 to be drawn longitudinally toward one another. This may cause the intermediate portion 146 of the anchor member 140 to bow radially outward toward the deployed configuration as shown in **FIG. 2****.** A loop or curve may be formed in the intermediate portion 146. The loop may be configured as a substantially smooth or continuous curve in the intermediate portion 146. In this manner, the intermediate portion 146 may be substantially free of straight segments and/or bends or angles formed between straight segments. This may aid in preventing damage to an inner surface of a body vessel which may be caused by contact with sharp bends or angles against the inner surface of the body vessel. In the deployed configuration, the anchor member 140 may extend in a nonlinear fashion between the proximal end 142 and the distal end 144 of the anchor member. The intermediate portion 146 of the anchor member 140 may be spaced radially outward away from the core member 110. In the deployed configuration, the outer radius of the anchor member 140 may be greater than the outer radius of the outer sleeve 120. This may aid in retaining the guide wire 100 in place within a body vessel as further described below.

In one example, the anchor member 140 may be biased toward a particular configuration. In other words, the anchor member 140 may be configured such that, in a relaxed condition, the anchor member tends to move toward the particular configuration. For example, the anchor member 140 may be biased toward the delivery configuration, the deployed configuration, or any other desired configuration. To that end, the anchor member 140 may be formed from a shape memory or superelastic material. The anchor member 140 may be formed from a shape memory or superelastic metal such as, for example, nitinol, stainless steel, copper-zinc-aluminum-nickel alloy, copper-aluminum-nickel alloy, or any other alloy which may include zinc, copper, gold, and/or iron. Additionally, or alternatively, the anchor member 140 may be formed from a shape memory polymer such as, for example, polyurethane, polyether ether ketone (PEEK), polyethylene, polyethylene terephthalate (PET), polyethylene oxide (PEO), polystyrene, or copolymers thereof. In one example, the anchor member 140 may be unbiased toward a particular configuration. In other words, the anchor member 140 may be configured such that, in a relaxed condition, the anchor member does not tend to move toward any particular configuration. To that end, the anchor member may be formed from a material that is substantially free of shape memory and/or superelastic properties.

In one example, the anchor member 140 may be biased toward the delivery configuration. In this manner, the anchor member 140 may urge the outer sleeve 120 proximally relative to the core member 110 toward the retracted position. In other words, the anchor member 140 may be biased toward a substantially linear configuration such that, with the anchor member 140 in a non-linear configuration, the anchor member may urge the outer sleeve 120 proximally relative to the core member 110 toward the retracted position. Because the anchor member 140 may be biased toward the delivery configuration, it may be unnecessary to position the anchor member within a tubular conduit to restrain the anchor member in the delivery configuration. In this manner, the guide wire 100 may be free of a tubular conduit such as a sheath positioned over the anchor member 140 in the delivery configuration. This may enable the guide wire 100 to have a reduced profile or outer diameter compared to a guide wire having an outer sheath.

The guide wire 100 may include two anchor members 140 as shown in FIGS. 1-2. Each anchor member 140 may be movable between the delivery configuration and the deployed configuration as described above. The anchor members 140 may be positioned opposite one another as described above such that the anchor members bow radially outward in opposite directions as shown in **FIG. 2****.** The anchor members 140 may be independent of one another. To that end, the anchor members 140 may be unattached to one another except at the proximal ends 142 via the outer sleeve 120 and at the distal ends 144 via the core member 110 and/or the tip 130. The intermediate portions 146 of the anchor members 140 may be unattached to one another. To that end, the guide wire 100 may be free of any structure extending between the intermediate portions 146 of the anchor members 140 (e.g., between adjacent intermediate portions). This may enable each anchor member 140 to bow radially outward independently of (e.g., without being constrained by) the radial position of another anchor member. Additionally, or alternatively, this may enable the anchor members 140 (e.g., the intermediate portions 146 of the anchor members) to move (e.g., by bending or flexing) toward and/or away from one another in a circumferential direction.

The intermediate portions 146 of the anchor members 140 may collectively define the outer diameter of the guide wire 100 at the longitudinal position of the anchor members. The outer diameter of the guide wire 100 at the longitudinal position of the anchor members 140 may be the distance between the outermost portion (e.g. the intermediate portion 146) of each anchor member. The outer diameter of the guide wire 100 at the longitudinal position of the anchor members may be greater in the deployed configuration than in the delivery configuration. In other words, the cross sectional area occupied by the anchor members 140 may be greater in the deployed configuration than in the delivery configuration. Additionally, or alternatively, the outer diameter of the guide wire 100 at the longitudinal position of the anchor members may be greater than the outer diameter of the outer sleeve 120.

The guide wire 100 may include six anchor members 140 as shown in **FIGS. 3-4****,** which illustrate a distal portion of the guide wire 100 in the delivery configuration and the deployed configuration, respectively. The anchor members 140 may be disposed circumferentially about the core member 110. The anchor members 140 may be spaced from one another by about 60 degrees. Each anchor member 140 may be configured as a flat wire or ribbon. Upon deployment, the anchor members 140 may bow radially outward as described above.

Increasing the number of anchor members 140 may increase the contact area of the anchor members with the surrounding body vessel wall. This may increase the force that may be required to pull the anchor members 140 through the body vessel. In other words, this may increase the holding force of the anchor members 140 to retain the guide wire 100 in place within the body vessel. Additionally, or alternatively, decreasing the number of anchor members 140 may decrease the force required to move the anchor members between the delivery configuration and the deployed configuration. The number of anchor members 140 may be selected to provide a sufficient holding force and a desirable tactile feel for the physician during deployment of the anchor members.

The retaining mechanism 150 may be positionable about the core member 110 near the proximal end 112 of the core member as shown in **FIGS. 1-2****.** The retaining mechanism 150 may be configured as a tubular member having a proximal end 152, a distal end 154, and a lumen extending longitudinally within the retaining mechanism between the proximal end and the distal end. The retaining mechanism 150 may be advanced distally over the proximal end 112 of the core member 110 such that the core member is received within the lumen of the retaining mechanism. The distal end 154 of the retaining mechanism 150 may be in abutting contact with the proximal end 122 of the outer sleeve 120. The retaining mechanism 150 may be unattached to the outer sleeve 120. Alternatively, the distal end 154 of the retaining mechanism 150 may be attached to the proximal end 122 of the outer sleeve 120. The retaining mechanism 150 may have a larger diameter than the outer sleeve 120. In this manner, the outer sleeve 120 may be substantially unable to move proximally along the core member 110 beyond the retaining mechanism 150.

The anchor members 140 may be biased toward the delivery configuration as described above. In this manner, the anchor members 140 may urge the outer sleeve 120 proximally relative to the core member 110 toward the retracted position. The outer sleeve 120 may be captured longitudinally between the anchor members 140 and the retaining mechanism 150. Advancing the retaining mechanism 150 distally over the core member 110 with sufficient force to overcome the urging force of the anchor members 140 may cause the outer sleeve 120 to move distally relative to the core member. This may cause the anchor members 140 to bow outward toward the deployed configuration as described above. Retracting the retaining mechanism 150 proximally over the core member 110 may enable the outer sleeve 120 to move proximally in response to the urging force of the anchor members 140. This may enable the anchor members 140 to move toward the delivery configuration as described above. The retaining mechanism 150 may be retracted proximally over the core member 110 and removed from the core member. This may enable delivery of an interventional medical device over the guide wire 100 as described below.

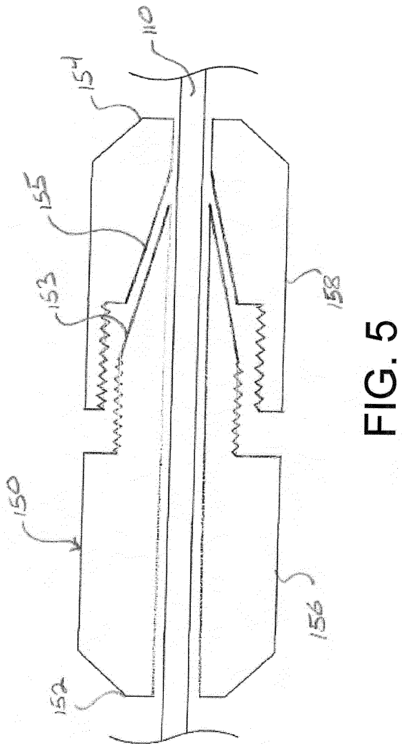

The retaining mechanism 150 may be configured to temporarily fix the longitudinal position of the outer sleeve 120 relative to the core member 110. To that end, the retaining mechanism 150 may be movable between a released configuration in which the core member 110 is capable of sliding longitudinally within the lumen of the retaining mechanism and an engaged configuration in which the core member is inhibited from sliding longitudinally within the lumen of the retaining mechanism. **FIG. 5** illustrates a longitudinal cross sectional view of one example of the retaining mechanism 150, which may be configured to function as a pin vise. A proximal portion 156 of the retaining mechanism 150 may be rotatable relative to a distal portion 158 of the retaining mechanism. Upon rotation of the proximal portion 156 relative to the distal portion 158 in a first rotational direction, the pin vise may engage the core member 110 received within the pin vise. In other words, rotation of the proximal portion 156 relative to the distal portion 158 in the first rotational direction may cause the pin vise to clamp or tighten around the core member 110. In this manner, the pin vise may frictionally engage the core member 110 to fix the pin vise in position longitudinally relative to the core member. Rotation of the proximal portion 156 relative to the distal portion 158 in a second rotational direction opposite the first rotational direction may cause the pin vise to loosen from the core member 110. In this manner, the pin vise may enable longitudinal movement of the core member 110 relative to the pin vise.

The proximal portion 156 of the retaining mechanism 150 may include a tapered protrusion 153 as shown in **FIG. 5****.** The distal portion 158 of the retaining mechanism 150 may include a tapered opening 155. The tapered protrusion 153 may include external threads configured to threadably engage with internal threads of the tapered opening 155. Rotation of the proximal portion 156 relative to the distal portion 158 may cause a corresponding longitudinal movement of the tapered protrusion 153 within the tapered opening 155. Threading the proximal portion 156 into the distal portion 158 (e.g., by rotating the proximal portion in the first rotational direction) may cause the tapered protrusion 153 to be engaged by the tapered opening 155. Such engagement may cause the tapered protrusion 153 to be compressed inward to frictionally engage the core member 110. Unthreading the proximal portion 156 from the distal portion 158 (e.g., by rotating the proximal portion in the second rotational direction) may cause the tapered protrusion to be disengaged from the tapered opening 155. Such disengagement may enable the tapered protrusion 153 to expand outward to disengage the core member 110. In other examples the proximal portion of the retaining mechanism may include a tapered opening, and the distal portion of the retaining mechanism may include a tapered protrusion.

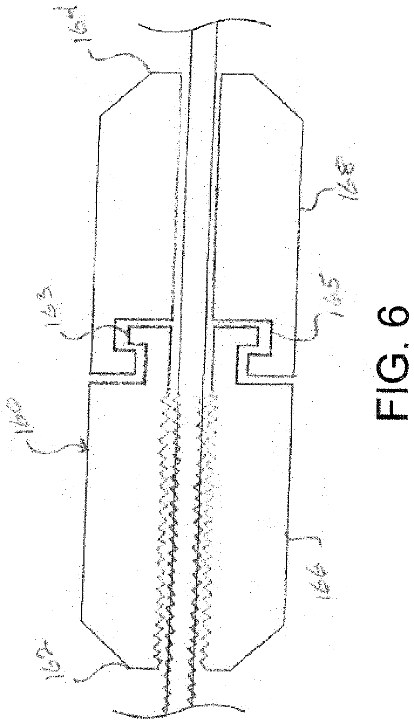

**FIG. 6** illustrates another example of a retaining mechanism 160. Although **FIGS. 1-2** show examples of the guide wire 100 including the retaining mechanism 150, the retaining mechanism 160 may be used instead of or in addition to the retaining mechanism 150. The retaining mechanism 160 may be configured as a tubular member having a proximal end 162, a distal end 164, and a lumen extending longitudinally within the retaining mechanism between the proximal end and the distal end as shown in **FIG. 6****.** The core member 110 may be received within the lumen of the retaining mechanism 160. The distal end 164 of the retaining mechanism 160 may be in abutting contact with and/or attached to the proximal end 122 of the outer sleeve 120. A proximal portion 166 of the retaining mechanism 160 may be rotatable relative to a distal portion 168 of the retaining mechanism. To that end, the proximal portion 166 may include a protrusion, which may be received within the distal portion 168. The protrusion of the proximal portion 166 may include an annular shoulder 163. The annular shoulder 163 may be received within an annular groove 165 of the distal portion 168 as shown in **FIG. 6****.** In this manner, the proximal portion 166 may be rotatable relative to the distal portion 168, while longitudinal movement of the proximal portion relative to the distal portion may be inhibited. In other examples, the distal portion may include an annular shoulder received within an annular groove of the proximal portion of the retaining mechanism.

The proximal portion 166 of the retaining mechanism 160 may include internal threads. A proximal portion of the core member 110 may include external threads to engage with the internal threads of the proximal portion 166 of the retaining mechanism 160. The retaining mechanism 160 may be threaded onto the proximal end 112 of the core member 110. Rotation of the proximal portion 166 of the retaining mechanism 160 relative to the core member 110 may cause longitudinal movement of the retaining mechanism relative to the core member. Such longitudinal movement of the retaining mechanism 160 relative the core member 110 may cause a corresponding longitudinal movement of the distal portion 168 of the retaining mechanism relative to the core member. This may cause longitudinal movement of the outer sleeve 120 relative to the core member 110 as described above. The threaded engagement between the retaining mechanism 160 and the core member 110 may enable precise control of the longitudinal position of the outer sleeve 120 relative to the core member. Additionally, or alternatively, the retaining mechanism 160 and/or the outer sleeve 120 may be inhibited from sliding longitudinally along the core member 110 without rotation of the proximal portion 166 of the retaining mechanism relative to the core member 110. This may aid in preventing inadvertent movement of the outer sleeve 120 between the retracted position and the advanced position and/or inadvertent movement of the anchor member 140 between the delivery configuration and the deployed configuration. In other examples, the distal portion 168 of the retaining mechanism 160 may be omitted, and the proximal portion 166 may abut the outer sleeve 120 as described above.

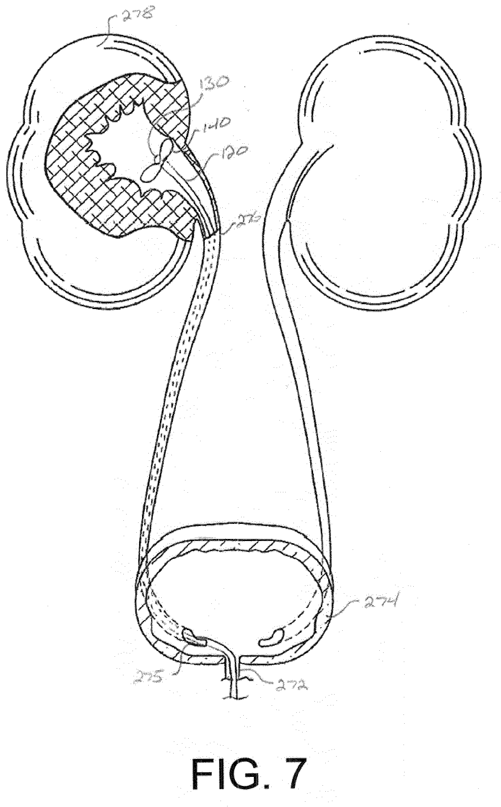

**FIG. 7** illustrates one example of placement of the guide wire 100 into a ureter of a patient. A cystoscope, or other suitable scope, may be introduced distally through a urethra 272 and into a bladder 274 in a conventional manner. The guide wire 100, with the anchor members 140 in the delivery configuration, may be introduced through the cystoscope and into the bladder 274. The guide wire 100 may be advanced through a ureteral orifice 275 and into the ureter 276. The guide wire 100 may be advanced distally within the ureter 276 and into a calyx of a kidney 278 as shown in **FIG. 7****.** With the distal end 114 of the core member 110 and/or the tip 130 of the guide wire 100 within the kidney 278, the anchor members 140 may be moved from the delivery configuration to the deployed configuration. For example, the retaining mechanism 150 may be advanced distally over the proximal end 112 of the core member 110 and into abutting contact with the proximal end 122 of the outer sleeve 120. The retaining mechanism 150 may be held in place while the proximal end 112 of the core member 110 is pulled proximally to move the outer sleeve 120 distally relative to the core member to move the anchor members 140 from the delivery configuration to the deployed configuration as described above with reference to **FIG. 5****.** Alternatively, the retaining mechanism 160 may be threaded onto the proximal end 112 of the core member 110 and distally into abutting contact with the proximal end 122 of the outer sleeve 120. The proximal portion 166 of the retaining mechanism 160 may be rotated relative to the core member 110 to move the outer sleeve 120 distally relative to the core member to move the anchor members 140 from the delivery configuration to the deployed configuration as described above with reference to **FIG. 6****.**

With the anchor members 140 in the deployed configuration, the retaining mechanism 150 may be moved from the released configuration to the engaged configuration to fix the outer sleeve 120 in place relative to the core member 110 as described above. This may aid in retaining the anchor members 140 in the deployed configuration (e.g., by preventing longitudinal movement of the outer sleeve 120 proximally relative to the core member 110). In the deployed configuration, the anchor members 140 may extend outward to an outer diameter that is greater than an inner diameter of the ureter 276 as shown in **FIG. 7****.** In this manner, the anchor members 140 may be substantially unable to move proximally through the ureter 276. This may aid in retaining the guide wire 100 in place within the ureter 276. The cystoscope may be retracted proximally over the guide wire 100 to remove the cystoscope from the urethra 272. The anchor members 140 may remain in the deployed configuration during retraction of the cystoscope to prevent inadvertent removal of the guide wire 100. To that end, the retaining mechanism 150 may be sized and shaped to pass through the lumen of the cystoscope as further described below so that the cystoscope may be withdrawn over the retaining mechanism. Preventing inadvertent removal of the guide wire 100 during retraction of the cystoscope may obviate the need to introduce a secondary or backup guide wire during the procedure, which may reduce the cost associated with the use of multiple guide wires. Additionally, or alternatively, preventing inadvertent removal of the guide wire 100 during retraction of the cystoscope may reduce the amount of lost time associated with replacing the guide wire after inadvertent removal.

With the cystoscope removed from the patient's body and the guide wire 100 in place within the ureter 276, the retaining mechanism 150 may be removed from the core member 110. The retaining mechanism 150 may be moved from the engaged configuration to the released configuration as described above to enable the retaining mechanism to move longitudinally relative to the core member 110. The retaining mechanism 150 may be retracted proximally over the core member 110 and removed from the proximal end 112 of the core member. Alternatively, the retaining mechanism 160 may be unthreaded from the core member 110 and removed from the proximal end 112 of the core member. Upon retraction of the retaining mechanism 150, the outer sleeve 120 may move from the advanced position to the retracted position, and/or the anchor members 140 may move from the deployed configuration to the delivery configuration. Additionally, or alternatively, the outer sleeve 120 may be held in place while the proximal end 112 of the core member 110 is pushed distally to move the outer sleeve proximally relative to the core member to move the anchor members 140 from the deployed configuration to the delivery configuration.

A ureteral stent, or other suitable interventional medical device, may be advanced over the guide wire 100 to place the ureteral stent within the ureter 276. The ureteral stent may have an inner diameter that is smaller than the outer diameter of the retaining mechanism 150. In this manner, advancing the ureteral stent over the guide wire 100 may be inhibited by the retaining mechanism 154 positioned on the core member 110 (e.g., because the retaining mechanism may be substantially unable to pass through the lumen of the ureteral stent). Removing the retaining mechanism 150 from the core member 110 as described above may enable the ureteral stent to be advanced over the guide wire 100. With the retaining mechanism 150 removed from the core member 110 and the anchor members 140 in the delivery configuration, the guide wire 100 may have a sufficiently small outer diameter to enable passage of the ureteral stent over the guide wire. With the ureteral stent in place within the ureter 276, the guide wire 100 may be withdrawn proximally from the patient's body through the ureteral stent.

The guide wire 100 and/or various components thereof may be sized and shaped for placement within any body lumen (e.g., the ureter as described above with reference to **FIG. 7****).** Accordingly, the dimensions described herein are exemplary, and the guide wire 100 and/or various components thereof may have any other dimensions suitable for an intended use. The outer sleeve 120 of the guide wire 100 may be sized and shaped to enable an interventional medical device to be introduced over the outer sleeve. In one example, the outer sleeve 120 may have an outer diameter of between about 0.4 mm (0.016 inches) and about 1 mm (0.038 inches). In one example, the outer sleeve 120 may have an outer diameter of between about 0.8 mm (0.032 inches) and about 1 mm (0.038 inches), typically about 0.9 mm (0.035 inches). This may enable a ureteral stent (e.g., a conventional 7 French (Fr) ureteral stent) to be introduced over the guide wire 100. The core member 110 may be sized and shaped to be received within the outer sleeve 120 as described above. To that end, the core member 110 may have an outer diameter that is less than or equal to an inner diameter of the outer sleeve 120. The tip 130 may have an outer diameter that is less than or equal to the outer diameter of the outer sleeve 120. The core member 110 and the outer sleeve 120 may have any suitable lengths. In one example, the core member 110 may have a length of between about 50 cm and about 260 cm. Additionally, or alternatively, the outer sleeve 120 may be between about 15 cm and about 30 cm shorter than the core member 110. To that end, the outer sleeve 120 may have a length of between about 20 cm and about 245 cm. In this manner, the outer sleeve 120 may have a sufficient length to enable direct manipulation of the proximal end 122, which may be positioned outside of the body during a medical procedure. Direct manipulation of the outer sleeve 120 may aid in navigating the guide wire 100 through a body vessel (e.g., by enabling pushing, pulling, and/or twisting of the outer sleeve).

The anchor members 140 may be positioned such that the outer diameter of the guide wire 100 at the longitudinal position of the anchor members in the delivery configuration may be less than or equal to the outer diameter of the outer sleeve 120. The outer diameter of the guide wire 100 at the longitudinal position of the anchor members 140 in the deployed configuration may depend on the length of the anchor members (e.g., the distance between the proximal ends 142 and the distal ends 144 of the anchor members). For example, longer anchor members may bow radially outward a greater radial distance than shorter anchor members in the deployed configuration. In one example, the anchor members 140 may have a length of between about 6 mm (0.25 inches) and about 64 mm (2.5 inches), typically between about 13 mm (0.5 inches) and about 51 mm (2 inches). In the deployed configuration, the anchor members 140 may bow radially outward to a radial distance of between about 3 mm (0.1 inches) and about 32 mm (1.25 inches), typically between about 5 mm (0.2 inches) and about 26 mm (1 inch). Additionally, or alternatively, the outer diameter of the guide wire 100 at the longitudinal position of the anchor members 140 in the deployed configuration may be between about 6 mm (0.232 inches) and about 65 mm (2.538 inches), typically between about 11 mm (0.435 inches) and about 52 mm (2.035 inches).

The retaining mechanism (e.g., the retaining mechanism 150 and/or the retaining mechanism 160) may be sized and shaped to be capable of passing through a lumen of a scope as described above. To that end, the retaining mechanism may be configured as a low profile retaining mechanism. For example, the retaining mechanism may have a French size of between about 3 Fr and about 12 Fr, typically between about 6 Fr and about 9 Fr. In other words, the retaining mechanism may have an outer diameter of between about 1 mm (0.039 inches) and about 4 mm (0.157 inches), typically between about 2 mm (0.079 inches) and about 3 mm (0.118 inches). This may enable the scope to be withdrawn over the guide wire 100, with the guide wire remaining in place within the body vessel and with the retaining mechanism engaged with the outer sleeve 120 to maintain the outer sleeve in the advanced position and the anchor members 140 in the deployed configuration as described above.

While various embodiments of the invention have been described, the invention is not to be restricted except in light of the attached claims and their equivalents. Drawings in the figures illustrating various embodiments are not necessarily to scale. Some drawings may have certain details magnified for emphasis, and any different numbers or proportions of parts should not be read as limiting unless so-designated in the present disclosure. Those skilled in the art will appreciate that embodiments not expressly illustrated herein may be practiced within the scope of the present invention, including those features described herein for different embodiments, which may be combined with each other and/or with currently-known or future-developed technologies while remaining within the scope of the claims presented herein. Moreover, the advantages described herein are not necessarily the only advantages of the invention and it is not necessarily expected that every embodiment of the invention will achieve all of the advantages described.

## Claims

1. A guide wire (100) comprising:
a core member (110) about a longitudinal axis of the guide wire and comprising a distal end (114);
an outer coil (120) disposed about the core member and comprising a proximal end (122) and a distal end (124) disposed proximal of the distal end of the core member, the outer coil extending longitudinally along a majority of a length of the guide wire and being longitudinally movable relative to the core member between a retracted position and an advanced position, the distal end of the outer coil being longitudinally spaced from the distal end of the core member by a greater distance in the retracted position than in the advanced position, wherein the outer coil (120) comprises an outer diameter of between about 0.8 mm (0.032 inches) and about 1.0 mm (0.038 inches);.
an anchor member (140) comprising an elongate wire extending longitudinally between the distal end (124) of the outer coil and the distal end (114) of the core member and comprising a proximal end (142) attached to the distal end of the outer coil and a distal end attached to the distal end of the core member, whereby, in response to longitudinal movement of the outer coil relative to the core member, the anchor member is movable between a delivery configuration and a deployed configuration, at least a portion of the anchor member being positioned radially away from the longitudinal axis of the guide wire by a greater distance in the deployed configuration than in the delivery configuration; and
a retaining mechanism (150) comprising a tubular member removably positionable about the core member and in abutting contact with a proximal end of the outer coil, the retaining mechanism being engageable with the core member to inhibit longitudinal movement of the outer coil proximally relative to the core member.

2. The guide wire (100) of claim 1, wherein the core member (110) comprises a solid wire.

3. The guide wire (100) of claim 1 or claim 2, wherein the retaining mechanism (150) comprises a larger outer diameter than the outer coil (120) and is removable from the core member (110) to enable passage of a tubular medical device over the guide wire.

4. The guide wire (100) of any one of the preceding claims, wherein the retaining mechanism (150) comprises a French size of between about 3 Fr and about 12 Fr.

5. The guide wire (100) of any one of the preceding claims, further comprising a tip (130) positioned at the distal end (114) of the core member (110), wherein the anchor member (140) is attached to the distal end of the core member via the tip.

6. The guide wire (100) of any one of the preceding claims, wherein the anchor member (140) comprises an intermediate portion (146) between the proximal end (142) and the distal end (144) of the anchor member, in response to movement of the outer coil (120) from the retracted position toward the advanced position, the proximal end of the anchor member and the distal end of the anchor member are moved longitudinally toward one another, and the intermediate portion of the anchor member bows radially outward away from the core member.

7. The guide wire (100) of any one of the preceding claims, wherein an outer diameter of the guide wire (100) at a longitudinal position of the anchor member (140) with the anchor member in the delivery configuration is less than or equal to an outer diameter of the outer coil (120), and the outer diameter of the guide wire at the longitudinal position of the anchor member with the anchor member in the deployed configuration is greater than the outer diameter of the outer coil.

8. The guide wire (100) of any one of the preceding claims, wherein the proximal end (142) of the anchor member (140) is fixedly attached to the distal end (124) of the outer coil (120) and the distal end of the anchor member (140) is fixedly attached to the distal end (114) of the core member (110).

9. The guide wire (100) of any one of the preceding claims, wherein the retaining mechanism (150) is movable between a released configuration in which the core member (110) is slidable within the retaining mechanism and an engaged configuration in which the returning mechanism is frictionally engaged with the core member to inhibit longitudinal movement of the outer coil (120) relative to the core member.

10. The guide wire (100) of any one of the preceding claims, wherein the anchor member (140) is biased toward the delivery configuration, whereby the outer coil (120) is biased toward the retracted position.

11. The guide wire (100) of any one of the preceding claims, wherein the anchor member (140) comprises a plurality of anchor members disposed about the core wire (110), and wherein in response to movement of the outer coil (120) from the retracted position toward the advanced position, the proximal end (142) of each anchor member and the distal end (144) of each anchor member are moved longitudinally toward one another, and a loop is formed in the intermediate portion of each anchor member.

12. The guide wire (100) of any one of the preceding claims, wherein the retaining mechanism (150) comprises a proximal portion (152) and a distal portion (154), and the proximal portion is rotatable relative to the distal portion to move the retaining mechanism between the released configuration and the engaged configuration.

13. The guide wire (100) of any one of the preceding claims, wherein the anchor member (140) comprises a plurality of anchor members disposed about the core wire (110), and further comprising a tip (130) positioned at the distal end (114) of the core member, wherein the distal end (144) of each anchor member (140) is attached to the distal end of the core member via the tip.

## Patentansprüche

1. Führungsdraht (100), umfassend:
ein Kernelement (110), das um eine Längsachse des Führungsdrahts angeordnet ist und ein distales Ende (114) umfasst;
eine Außenspirale (120), die um das Kernelement herum angeordnet ist und ein proximales Ende (122) und ein distales Ende (124), das proximal vom distalen Ende des Kernelements angeordnet ist, umfasst, wobei sich die Außenspirale längs entlang eines größten Teils einer Länge des Führungsdrahts erstreckt und bezüglich des Kernelements längs zwischen einer zurückgezogenen Position und einer vorgeschobenen Position bewegbar ist, wobei das distale Ende der Außenspirale in der zurückgezogenen Position längs in einem größeren Abstand vom distalen Ende des Kernelements angeordnet ist als in der vorgeschobenen Position, wobei die Außenspirale (120) einen Außendurchmesser zwischen ungefähr 0,8 mm (0,032 Zoll) und ungefähr 1,0 mm (0,038 Zoll) umfasst;
ein Verankerungsmittel (140), das einen langgestreckten Draht umfasst, der sich längs zwischen dem distalen Ende (124) der Außenspirale und dem distalen Ende (114) des Kernelements erstreckt und ein proximales Ende (142), das am distalen Ende der Außenspirale befestigt ist, und ein distales Ende, das am distalen Ende des Kernelements angeordnet ist, umfasst, wobei das Verankerungsmittel als Reaktion auf eine Längsbewegung der Außenspirale bezüglich des Kernelements zwischen einer Freisetzungskonfiguration und einer Einsatzkonfiguration bewegbar ist, wobei zumindest ein Teil des Verankerungselements in der Einsatzkonfiguration in einem größeren Abstand radial von der Längsachse des Führungsdrahts entfernt angeordnet ist als in der Freisetzungskonfiguration; und
einen Haltemechanismus (150), der ein röhrenförmiges Element umfasst, das entfernbar um das Kernelement herum und in anstoßendem Kontakt mit einem proximalen Ende der Außenspirale angeordnet werden kann, wobei der Haltemechanismus mit dem Kernelement in Eingriff gebracht werden kann, um eine Längsbewegung der Außenspirale bezüglich des Kernelements zu unterdrücken.

2. Führungsdraht (100) nach Anspruch 1, wobei das Kernelement (110) einen festen Draht umfasst.

3. Führungsdraht (100) nach Anspruch 1 oder Anspruch 2, wobei der Haltemechanismus (150) einen größeren Außendurchmesser als die Außenspirale (120) umfasst und vom Kernelement (110) entfernt werden kann, damit eine röhrenförmige medizinische Vorrichtung über den Führungsdraht geführt werden kann.

4. Führungsdraht (100) nach einem der vorhergehenden Ansprüche, wobei der Haltemechanismus (150) eine Größe in French zwischen ungefähr 3 Fr und ungefähr 12 Fr umfasst.

5. Führungsdraht (100) nach einem der vorhergehenden Ansprüche, ferner umfassend eine Spitze (130), die am distalen Ende (114) des Kernelements (110) angeordnet ist, wobei das Verankerungselement (140) über die Spitze am distalen Ende des Kernelements befestigt ist.

6. Führungsdraht (100) nach einem der vorhergehenden Ansprüche, wobei das Verankerungselement (140) einen Zwischenabschnitt (146) zwischen dem proximalen Ende (142) und dem distalen Ende (144) des Verankerungselements umfasst, wobei, als Reaktion auf eine Bewegung der Außenspirale (120) aus der zurückgezogenen Position in die vorgeschobene Position, das proximale Ende des Verankerungselements und das distale Ende des Verankerungselements längs zueinander bewegt werden, und sich der Zwischenabschnitt des Verankerungselements vom Kernelement weg radial nach außen biegt.

7. Führungsdraht (100) nach einem der vorhergehenden Ansprüche, wobei ein Außendurchmesser des Führungsdrahts (100) in einer Längsposition des Verankerungselements (140) mit dem Verankerungselement in der Freisetzungskonfiguration gleich oder kleiner als ein Außendurchmesser der Außenspirale (120) ist, und wobei der Außendurchmesser des Führungsdrahts in der Längsposition des Verankerungselements mit dem Verankerungselement in der Einsatzkonfiguration größer als der Außendurchmesser der Außenspirale ist.

8. Führungsdraht (100) nach einem der vorhergehenden Ansprüche, wobei das proximale Ende (142) des Verankerungselements (140) fest am distalen Ende (124) der Außenspirale (120) befestigt ist und das distale Ende des Verankerungselements (140) fest am distalen Ende (114) des Kernelements (110) befestigt ist.

9. Führungsdraht (100) nach einem der vorhergehenden Ansprüche, wobei der Haltemechanismus (150) zwischen einer Freigabekonfiguration, in der das Kernelement (110) im Haltemechanismus gleiten kann, und einer Eingriffskonfiguration, in der der Rückführungsmechanismus mit dem Kernelement in reibschlüssigem Eingriff ist, bewegt werden kann, um eine Längsbewegung der Außenspirale (120) bezüglich des Kernelements zu unterdrücken.

10. Führungsdraht (100) nach einem der vorhergehenden Ansprüche, wobei das Verankerungselement (140) in die Freisetzungskonfiguration vorgespannt ist, wobei die Außenspirale (120) in die zurückgezogene Position vorgespannt ist.

11. Führungsdraht (100) nach einem der vorhergehenden Ansprüche, wobei das Verankerungselement (140) eine Vielzahl von Verankerungselementen umfasst, die um den Kerndraht (110) herum angeordnet sind, und wobei das proximale Ende (142) jedes Verankerungselements und das distale Ende (144) jedes Verankerungselements als Reaktion auf eine Bewegung der Außenspirale (120) aus der zurückgezogenen Position in die vorgeschobene Position längs zueinander bewegt werden, und eine Schlaufe im Zwischenabschnitt jedes Verankerungselements gebildet wird.

12. Führungsdraht (100) nach einem der vorhergehenden Ansprüche, wobei der Haltemechanismus (150) einen proximalen Abschnitt (152) und einen distalen Abschnitt (154) umfasst, und der proximale Abschnitt bezüglich des distalen Abschnitts bewegbar ist, um den Haltemechanismus zwischen der Freigabekonfiguration und der Eingriffskonfiguration zu bewegen.

13. Führungsdraht (100) nach einem der vorhergehenden Ansprüche, wobei das Verankerungselement (140) eine Vielzahl von Verankerungselementen umfasst, die um den Kerndraht (110) herum angeordnet sind, und ferner umfassend eine Spitze (130), die am distalen Ende (114) des Kernelements angeordnet ist, wobei das distale Ende (144) jedes Verankerungselements (140) über die Spitze am distalen Ende des Kernelements befestigt ist.

## Revendications

1. Fil-guide (100) comportant :
un élément (110) central autour d'un axe longitudinal du fil-guide et comportant une extrémité (114) distale ;
une spirale (120) extérieure disposée autour de l'élément central et comportant une extrémité (122) proximale et une extrémité (124) distale disposée de manière proximale par rapport à l'extrémité distale de l'élément central, la spirale extérieure s'étendant longitudinalement sur une plus grande partie d'une longueur du fil-guide et étant mobile longitudinalement par rapport à l'élément central entre une position rétractée et une position avancée, l'extrémité distale de la spirale extérieure étant séparée longitudinalement de l'extrémité distale de l'élément central par une distance plus grande dans la position rétractée que dans la position avancée, dans lequel la spirale (120) extérieure comporte un diamètre extérieur compris entre environ 0,8 mm (0,032 pouce) et environ 1,0 mm (0,038 pouce) ;
un élément (140) d'ancrage comportant un fil allongé qui s'étend longitudinalement entre l'extrémité (124) distale de la spirale extérieure et l'extrémité (114) distale de l'élément central et comportant une extrémité (142) proximale fixée à l'extrémité distale de la spirale extérieure et une extrémité distale fixée à l'extrémité distale de l'élément central, lequel élément d'ancrage, en réponse à un déplacement longitudinal de la spirale extérieure par rapport à l'élément central, est mobile entre une configuration de pose et une configuration déployée, au moins une partie de l'élément d'ancrage étant positionnée radialement à distance de l'axe longitudinal du fil-guide à une distance plus grande dans la configuration déployée que dans la configuration de pose ; et
un mécanisme (150) de retenue comportant un élément tubulaire pouvant être positionné de manière amovible autour de l'élément central et venir en contact par butée avec une extrémité proximale de la spirale extérieure, le mécanisme de retenue pouvant être mis en prise avec l'élément central pour empêcher le déplacement longitudinal de la spirale extérieure dans le sens proximal par rapport à l'élément central.

2. Fil-guide (100) selon la revendication 1, dans lequel l'élément (110) central comporte un fil plein.

3. Fil-guide (100) selon la revendication 1 ou la revendication 2, dans lequel le mécanisme (150) de retenue comporte un diamètre extérieur plus grand que la spirale (120) extérieure et peut être retiré de l'élément (110) central pour permettre le passage d'un dispositif médical tubulaire sur le fil-guide.

4. Fil-guide (100) selon l'une quelconque des revendications précédentes, dans lequel le mécanisme (150) de retenue comporte une taille en French comprise entre environ 3 Fr et environ 12 Fr.

5. Fil-guide (100) selon l'une quelconque des revendications précédentes, comportant en outre un bout (130) positionné au niveau de l'extrémité (114) distale de l'élément (110) central, dans lequel l'élément (140) d'ancrage est fixé à l'extrémité distale de l'élément central par le biais du bout.

6. Fil-guide (100) selon l'une quelconque des revendications précédentes, dans lequel l'élément (140) d'ancrage comporte une partie (146) intermédiaire entre l'extrémité (142) proximale et l'extrémité (144) distale de l'élément d'ancrage, en réponse au déplacement de la spirale (120) extérieure de la position rétractée vers la position avancée, l'extrémité proximale de l'élément d'ancrage et l'extrémité distale de l'élément d'ancrage sont déplacées longitudinalement l'une vers l'autre, et la partie intermédiaire de l'élément d'ancrage s'arque radialement vers l'extérieur à distance de l'élément central.

7. Fil-guide (100) selon l'une quelconque des revendications précédentes, dans lequel un diamètre extérieur du fil-guide (100) au niveau d'une position longitudinale de l'élément (140) d'ancrage avec l'élément d'ancrage dans la configuration de pose est inférieur ou égal à un diamètre extérieur de la spirale (120) extérieure, et le diamètre extérieur du fil-guide au niveau de la position longitudinale de l'élément d'ancrage avec l'élément d'ancrage dans la configuration déployée est supérieur au diamètre extérieur de la spirale extérieure.

8. Fil-guide (100) selon l'une quelconque des revendications précédentes, dans lequel l'extrémité (142) proximale de l'élément (140) d'ancrage est fixée à demeure à l'extrémité (124) distale de la spirale (120) extérieure et l'extrémité distale de l'élément (140) d'ancrage est fixée à demeure à l'extrémité (114) distale de l'élément (110) central.

9. Fil-guide (100) selon l'une quelconque des revendications précédentes, dans lequel le mécanisme (150) de retenue est mobile entre une configuration libérée dans laquelle l'élément (110) central est coulissant à l'intérieur du mécanisme de retenue et une configuration mise en prise dans laquelle le mécanisme de retour est mis en prise par frottement avec l'élément central pour empêcher un déplacement longitudinal de la spirale (120) extérieure par rapport à l'élément central.

10. Fil-guide (100) selon l'une quelconque des revendications précédentes, dans lequel l'élément (140) d'ancrage est sollicité vers la configuration de pose, laquelle spirale (120) extérieure est sollicitée vers la position rétractée.

11. Fil-guide (100) selon l'une quelconque des revendications précédentes, dans lequel l'élément (140) d'ancrage comporte une pluralité d'éléments d'ancrage disposés autour du fil (110) central, et dans lequel en réponse au déplacement de la spirale (120) extérieure de la position rétractée vers la position avancée, l'extrémité (142) proximale de chaque élément d'ancrage et l'extrémité (144) distale de chaque élément d'ancrage sont déplacées longitudinalement l'une vers l'autre, et une boucle est formée dans la partie intermédiaire de chaque élément d'ancrage.

12. Fil-guide (100) selon l'une quelconque des revendications précédentes, dans lequel le mécanisme (150) de retenue comporte une partie (152) proximale et une partie (154) distale, et la partie proximale est rotative par rapport à la partie distale pour déplacer le mécanisme de retenue entre la configuration libérée et la configuration mise en prise.

13. Fil-guide (100) selon l'une quelconque des revendications précédentes, dans lequel l'élément (140) d'ancrage comporte une pluralité d'éléments d'ancrage disposés autour du fil (110) central, et comportant en outre un bout (130) positionné au niveau de l'extrémité (114) distale de l'élément central, dans lequel l'extrémité (144) distale de chaque élément (140) d'ancrage est fixée au niveau de l'extrémité distale de l'élément central par le biais du bout.
